(19) Europäisches Patentamt
European Patent Office
Office européen des brevets

(11) Publication number: 0 004 794
B1

(12) # EUROPEAN PATENT SPECIFICATION

(45) Date of publication of patent specification: 28.04.82

(51) Int. Cl.³: C 07 D 223/16, A 61 K 31/55

(21) Application number: 79300601.6

(22) Date of filing: 11.04.79

(54) **Substituted benzazepines, processes for preparing them and pharmaceutical compositions containing them.**

(30) Priority: 12.04.78 US 895700

(43) Date of publication of application: 17.10.79 Bulletin 79/21

(45) Publication of the grant of the patent: 28.04.82 Bulletin 82/17

(84) Designated Contracting States: BE CH DE FR GB IT LU NL SE

(56) References cited:
CH - A - 555 831
LU - A - 78 513
LU - A - 78 873

(73) Proprietor: SMITHKLINE CORPORATION
1500 Spring Garden Street P.O. Box 7929
Philadelphia Pennsylvania 19101 (US)

(72) Inventor: Weinstock, Joseph
1234 Pothouse Road
Phoenixville Pennsylvania 19405 (US)

(74) Representative: Hargreaves, Gerald Henry, Dr. et al,
P.O.Box 39
Welwyn Garden City Hertfordshire AL7 1EY (GB)

EP 0 004 794 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Substituted benzazepines, processes for preparing them and pharmaceutical compositions containing them

This invention concerns 1-phenyl-2,3,4,5-tetrahydro-1H-3-benzazepines having four substituents on the benz-ring, processes for their preparation and pharmaceutical compositions containing them. These compounds have dopaminergic activity and especially antihypertensive activity.

Swiss Patent Specification 555,831 describes generically benz-trisubstituted benzazepines but no dopaminergic activity. U.S. Patent Specification 4,011,319 describes peripheral dopaminergic activity for certain benz-mono and disubstituted benzazepines. Certain substituted benzazepines having at least three substituents on the benz-ring and exhibiting dopaminergic activity are described in Luxembourg Patent 78513.

The compounds of this invention are of formula:

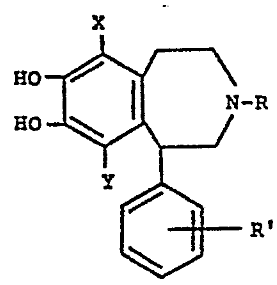

I

in which X and Y, which are the same or different, are each chloro, fluoro or a lower alkyl of 1—4 carbons, especially methyl, ethyl, propyl or butyl; R is hydrogen, methyl, ethyl, allyl, dimethylallyl, thenyl or furylmethyl; and R′ is hydrogen or one or two methoxy, hydroxy, acetoxy, halo, trifluoromethyl, methyl or methylthio substituents.

Preferred compounds are those of formula I in which R is hydrogen. Another group of compounds of formula I has X and Y each chloro or methyl, R hydrogen and R′ hydrogen, *m*-hydroxy, *p*-hydroxy or *m*-methyl.

The present invention also includes pharmaceutically acceptable acid addition salts of the compounds of formula I. These salts can be prepared by methods well known to the art, and can be formed with either inorganic or organic acids, for example maleic, fumaric, benzoic, ascorbic, pamoic, succinic, bis methylenesalicyclic, methanesulfonic, ethanedisulfonic, acetic, oxalic, propionic, tartaric, salicylic, citric, gluconic, aspartic, stearic, palmitic, itaconic, glycolic, p-aminobenzoic, glutamic, benzenesulfonic, hydrochloric, hydrobromic, sulfuric, cyclohexylsulfamic, phosphoric and nitric acids. The hydrohalic and methanesulfonic acid salts are particularly preferred.

Also includes in this invention are bioprecursors of the compounds of formula I and derivatives of the compounds of formula I which are converted *in vivo* into the compounds of this invention. Such may be the O-lower alkanoyl derivatives, such as at the 7,8-dihydroxy groups of the compounds of formula I derived from lower alkanoic acids of 2—7 carbons for example diacetoxy, di-isobutyrylkoxy, di-isovaleryloxy, dipropionyloxy derivatives. Whenever R′ is hydroxy a similar alkanoyl group can also be introduced at that position.

The compounds of formula I can be present as diasteroisomers which can be resolved into *d, l* optical isomers. Resolution of the optical isomers can be conveniently accomplished by fractional crystallization of their salts with optically active acids from appropriate solvents.

Unless otherwise specified herein or in the claims, all isomers, whether separated or mixtures thereof, are included within this invention. Where isomers are separated, the desired pharmacological activity will usually predominate in one of the isomers.

The compounds of formula I can be prepared from intermediates of the formula:

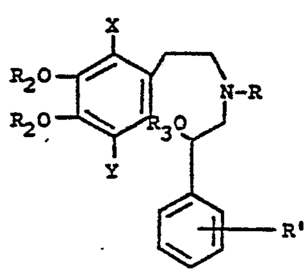

II

(in which X, Y, R and R′ are as hereinbefore defined; both $R_2$ groups are benzyl, ($C_1$—$C_7$) lower alkyl especially methyl or together, methylene; $R_3$ is hydrogen or a functionally equivalent group for generating with the attached oxygen atom a carbonium ion at the $\alpha$-position of the phenethanolamine moiety) by an intramolecular cyclization effected using a cyclization agent, for example sulfuric acid

alone, or trifluoroacetic acid, polyphosphoric acid or a similar dehydrating agent mixed with a solvent.

The intermediates of formula II above are conveniently prepared by heating equimolar amounts of an appropriately substituted styrene oxide with an appropriately substituted 3,4-dialkoxyphenethylamine, either alone or in an inert organic solvent, for example tetrahydrofuran. Preferably the heating is effected on a steam bath or at reflux temperature for from 12 to 24 hours. The required styrene oxide is conveniently prepared by reaction of the ylide derivatives produced by reacting sodium hydride and trimethylsulfonium iodide with the appropriately substituted benzaldehyde. Other preparations of the intermediates of formula II will be apparent from the Examples presented hereafter.

The 2,5-disubstituted-3,4-dialkoxyphenethylamines used to prepare the intermediates of formula II are not reported in the literature. The methods of the prior art for preparing 1-phenyl-2,3,4,5-tetrahydro-1H-3-benzazepines therefore cannot be used to prepare compounds of the present invention without access to the proper starting materials.

The tetrasubstituted phenethylamines can be prepared from catechol by inserting halogens in the ortho-positions or by inserting methyl groups by a Mannich reaction-reduction. After O-methylation, the amino ethyl group can be inserted by chloromethylation, cyanide-reduction sequence of steps, or a Mannich reaction-reduction sequence, if appropriate. Protective ether groups can be added whenever appropriate. The intermediates of formula II can then be prepared from the phenethylamines as described above.

In certain cases, such as when the cyclization proceeds with difficulty or when the starting material is not easily prepared, it can be advantageous to insert the 6,9-substituents directly into the formed 7,8-dihydroxy-1-phenyl-2,3,4,5-tetrahydro-1H-3-benzazepine.

One example of this direct insertion is 6,9-halogenation, with interfering groups protected. Also, the catecholic 7,8-dihydroxy system can be oxidized, for example by hypohalite or DDQ (2,3-dichloro-5,6-dicyano-1,4-benzoquinone), to the 7,8-quinone. Hydrochloric acid is then added to the sensitive 7,8-quinone system. Alternatively, combinations of the total synthetic and insertion methods can be used.

The compounds of this invention can therefore be prepared by one of two chemical methods. First by reacting an appropriate 6,9-disubstituted-7,8-dihydroxy-1-phenyl-2,3,4,5-tetrahydro-1H-3-benzazepine which has O-protective groups, especially ($C_1$—$C_7$) lower alkyl or benzyl ethers at the 7,8-positions and, if present, phenolic hydroxy groups, with a dealkylating agent. The dealkylating agent can be a hydrohalic acid, such as hydrobromic or hydriodic acid, or boron tribromide. The O-protected starting materials are preferably dissolved in a solvent in which they are at least partially soluble, the dealkylation agent is added, and the mixture is usually heated at reflux for from 1—6 hours.

The second method of preparing the compounds of the invention where Y is chloro involves the addition of hydrochloric acid to an appropriate 7,8-dione of the formula:

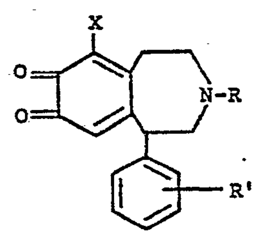

III

in which X, R and R′ are as defined for formula I. Hydrochloric acid is preferably reacted with the appropriate 7,8-quinone of formula III in an inert organic solvent at ambient temperature. Usually the reaction will be effected in a ($C_1$—$C_4$) lower alkanol such as methanol, ethanol or isopropanol at room temperature. The hydrohalic addition reaction can be carried out as a distinct step or directly in the reaction mixture in which 7,8-quinone is prepared by oxidation of the 7,8-dihydroxy compound, for example in the hypochlorite reaction described in the Examples. In each process the reaction can be concluded by neutralization to obtain the free base and subsequent formation of salt or ester derivatives known to the art.

The compounds of formula I were R is methyl, ethyl, allyl or other defined substituents are conveniently prepared from, for example, benzazepines having ether groups for protection by standard N-alkylation method, such as using reactive lower alkyl halides, acylation-reduction or, for the methyl compounds, formic acid/formaldehyde of formaldehyde/catalytic hydrogenation. Reaction of the resulting products with a dealkylation agent e.g. boron tribromide then gives the corresponding hydroxy-substituted benzazepines.

To prepare the O-alkanoyl derivatives of the compounds of formula I, the corresponding 3-benzyl-7,8-dihydroxy-3-benzazepine (obtained by N-alkylation of the corresponding hydroxybenzazepine with benzyl bromide in the presence of potassium carbonate) can be reacted with the appropriate alkanoic acid anhydride or chloride, for example acetic anhydride, and the resulting alkanoyloxy-substituted benzazepine can then be hydrogenated in the presence of palladium-on-carbon to remove the protecting benzyl group. The dialkanoyloxy derivatives, for example the important 7,8-diacetoxy

compounds, can also be prepared by direct O-acylation of the appropriate 7,8-dihydroxy-1-phenyl-2,3,4,5-tetrahydro-1H-3-benzazepine hydrobromide in trifluoroacetic acid at ambient temperature using the appropriate anhydride or halide.

The compounds of this invention stimulate peripheral dopamine receptors, for example they increase renal blood flow and they have as an end result hypotensive activity. This renal vasodilator activity of the benzazepine compounds of formula I has been measured in an anesthetized dog. In this pharmacological procedure, a test compound is administered at progressively increasing (3-fold) infusion rates beginning at 0.1 mcg/kg/min up to 810 mcg/kg/min for 5 minutes each to anesthetized normotensive dogs, and the following parameters are measured: renal artery blood flow, iliac artery blood flow, arterial blood pressure and heart rate. Results are reported as a percent change, increase or decrease, at the time of peak response (from pre-drug controls), and for a significant effect renal blood flow (increase) and renal vascular resistance (decrease) should be approximately 10% or greater. The effect on renal vascular resistance can be calculated from any change in renal blood flow and arterial blood pressure. To confirm the mechanism of action, representative active renal vasodilator compounds are checked for blockade by bulbocapnine which is known to be a specific blocker of renal dopamine receptors. 6,9-Dichloro-7,8-dihydroxy-1-phenyl-2,3,4,5-tetrahydro-1H-3-benzazepine, used in the form of its hydrochloride salt, tested by i.v. infusion as described above, produced an $ED_{15}$ of 3.5 mcg/kg with little direct effect on systemic blood pressure in normotensive animals. For comparison, 6,9-dibromo-7,8-dihydroxy-1-phenyl-2,3,4,5-tetrahydro-1H-3-benzazepine hydrochloride has an $ED_{15}$ of 634 mcg/kg in 4 dogs. $ED_{15}$ is the cumulative dose by infusion which produces a 15% decrease in renal vascular resistance

$$(R = \frac{\text{B.P. in mm/hg}}{\text{B.F. ml/min}}).$$

The preferred compounds of this invention, for example, the 6,9-dichloro compound mentioned above, also have dampened dopaminergic activity at central receptor sites as measured by testing for anti-parkinsonism activity using a modified standard pharmacological test procedure reported by Ungerstedt *et al.*, in *Brain Research* 24, 1970, 485—493. This procedure is based on a drug-induced rotation of rats having extensive unilateral lesions of the substantia nigra. Briefly, the test consists of the quantitative recording of rotational behavior in rats in which 6-hydroxy-dopamine lesions of the nigrostriatal dopamine system have been produced. A unilateral brain lesion in the left substantia nigra causes the dopamine receptor in the left caudate to become hypersensitive following the resulting degeneration of the nigral cell bodies. These lesions destroy the source of the neurotransmitter dopamine in the caudate but they leave the caudate cell bodies and their dopamine receptors intact. Activation of these receptors by drugs which produce contralateral rotation, with respect to the lesioned side of the brain, is used as a measure of central dopaminergic activity of the drug.

Compound which are known to be clinically effective in controlling parkinsonism, for example L-dopa and apomorphine, are also effective in this rat-turning model. These compounds directly activate the central dopamine receptors and they cause contralateral rotation of the lesioned rat.

Rotational activity of a compound is the ability of the compound to produce 500 contralateral rotations during a two-hour period after administration, usually intraperitoneally. The dose corresponding to 500 contralateral rotations per two hours is the $RD_{500}$ value.

6,9-Dichloro-7,8-dihydroxy-1-phenyl-2,3,4,5-tetrahydro-1H-3-benzazepine hydrochloride is not sufficiently active in this test at 10 mg/kg i.p. to give a value for $RD_{500}$. In fact, it gave 496 $\pm$ 121 rotations. In comparison 6-chloro-7,8-dihydroxy-1-phenyl-2,3,4,5-tetrahydro-1H-3-benzazepine hydrobromide had a $RD_{500}$ of 0.3 mg/kg. Insertion of a fourth substituent at position 9 therefore produced a compound 30 times less active at central receptors than its 6-chloro-7,8-dihydroxy analogue. 6,9-Dichloro-7,8-dihydroxy-1-phenyl-2,3,4,5-tetrahydro-1H-3-benzazepine hydrochloride shows indications in other test procedures that it has low pressor activity. The compounds of the invention therefore show a specific peripheral dopaminergic activity.

A specific and preferred group of compounds is therefore these of formula I in which X and Y are each chloro, fluoro, methyl, ethyl or propyl, with R being hydrogen and R′ being hydrogen or especially *p*-hydroxyl. These have potent, specific peripheral dopaminergic activity giving increased renal blood flow.

N-Substituents in the compounds of formula I has been found to increase central dopaminergic activity.

The present invention includes pharmaceutical compositions having dopaminergic activity, and especially anti-hypertensive activity. The compositions of the invention can be prepared in conventional dosage unit forms from a compound of the invention, and a pharmaceutically acceptable carrier according to accepted procedures using a non-toxic amount of the compound of the invention sufficient to produce the desired pharmacodynamic activity in a subject, animal or human. Preferably, the compositions contain a compound of the invention in an active but non-toxic amount of from about 15 mg to about 500 mg per dosage unit, preferably from 25—200 mg, but this quantity depends on the

specific biological activity desired, the activity of the compound of the invention, the route of administration, and the condition of patient.

The pharmaceutical carrier can be solid or liquid. Examples of solid carriers are lactose, terra alba, sucrose, talc, gelatin, agar, pectin, acacia, magnesium stearate, stearic acid, and the like. Examples of liquid carriers are syrup, peanut oil, olive oil, water and the like. Similarly the carrier or diluent can include time delay material known to the art, for example glyceryl monostearate or glyceryl distearate, alone or with a wax.

A wide variety of pharmaceutical forms can be employed. Thus, if a solid carrier for oral administration is used, the composition can be tableted, placed in a hard gelatin capsule in powder or pellet form to be in the form of a troche or lozenge. The amount of solid carrier can be varied widely but preferably it is from about 25 mg to about 1 g. If a liquid carrier is used, the composition can be in the form of a syrup, emulsion, soft gelatin capsule, sterile injectable liquid such as an ampoule, or as an aqueous or nonaqueous liquid suspension.

The pharmaceutical compositions can be made following conventional techniques of the pharmaceutical chemist and involve mixing, granulating and compressing, when necessary, or variously mixing and dissolving the ingredients as appropriate to give the desired composition.

A dopaminergic effect can be obtained from the compositions of this invention by administering internally to a subject a dosage unit of a compound of the invention, usually combined with a pharmaceutical carrier, in a non-toxic amount sufficient to produce the desired effect. Any route of administration can be used which effectively transports the active compound to the dopamine receptors which are to be stimulated, for example orally or parenterally, the oral route being preferred. Advantageously, equal doses will be administered several times a day, for example two or three times a day, with a preferred daily dosage regimen being from about 30 mg to about 2 g. When administration is effected as described above, a dopaminergic effect, especially an anti-hypertensive effect is obtained due to increased renal blood flow with a minimum of side effects.

The following Examples illustrate the compounds of this invention. All temperatures are in degrees Centigrade. Other variations of these Examples will be obvious to those skilled in the art.

Example 1

A mixture of 69 g (0.5 mole) of 3,6-dimethyl catechol [J. Org. Chem. *29*: 2640 (1964)], 200 ml (266 g, 2.11 moles) of dimethyl sulfate, and 700 ml of 10% sodium hydroxide is refluxed under nitrogen for 1 hour. Then, an additional 700 ml of 10% sodium hydroxide is added, and the mixture is stirred at 25° for 18 hours. The reaction mixture is diluted with water, extracted twice with ether, and the ether is washed with water and dried. Concentration gives 49 g (59%) of 3,6-dimethylveratrole.

A solution of 43 g (0.20 mole) of the chloromethyl compound in 220 ml of dimethylsulfoxide was treated with 11.36 g (0.23 mole) of sodium cyanide and the mixture is stirred at 50° for 1 hour. The reaction mixture is added to 150 ml of ice, and then diluted with 650 ml of cold water. The product is extracted with ether which was washed with water, dried, and then concentrated under vacuum to give 2,5-dimethyl-3,4-dimethoxybenzylcyanide.

A mixture of 18.3 g (0.0865 mole) of the nitrile, 1.6 g of Raney nickel catalyst and 170 ml of saturated anhydrous methanolic ammonia is hydrogenated at 50°, kg/cm$^2$ of hydrogen gas until hydrogen uptake stopped (about 6 hours). Removal of the catalyst by filtration followed by evaporation gives the 2-(2,5-dimethyl-3,4-dimethoxyphenyl)ethylamine which can be used without further purification.

The phenethylamine (25.7 g, .12 mole) is heated to 115° in an oil bath. Styrene oxide (14.4 g, .12 mole) is added and the reaction heated for 1 hour. After cooling to 30°, 2:1 petroleum ether/acetone is added to dissolve the oil; N-[2-hydroxy-2-phenylethyl)]-N-[2-(2′,5′-dimethyl-3′,4′-dimethoxyphenyl)ethyl]amine. The product can be used as the oil or after crystallizing from the ether/acetone mixture.

The hydroxyphenethylamine (10 g) is dissolved in 40 ml of trifluoroacetic acid and 3 ml of concentrated sulfuric acid are added. The reaction mixture is refluxed for 2 hours. After cooling, most of the trifluoroacetic acid is stripped off and the residue is poured into water. The water is made basic with 10% sodium hydroxide and extracted with ether twice. The dried ether is evaporated to leave a solid; 6,9-dimethyl-7,8-dimethoxy-1-phenyl-2,3,4,5-tetrahydro-1H-3-benzazepine.

The dimethoxy derivative (5 g) is converted into 6,9-methyl-7,8-dihydroxy-1-phenyl-2,3,4,5-tetrahydro-1H-3-benzazepine hydrobromide using boron tribromide in methylene chloride at −15°. After standing at room temperature for 3 hours, the solvent is stripped. The residue is treated with cold methanol. Stripping gives the desired compound.

Example 2

A slurry of 29.1 g (0.1 mole) of 7,8-dihydroxy-1-phenyl-2,3,4,5-tetrahydro-1H-3-benzazepine hydrochloride in 470 ml of 9N hydrochloric acid at 28° was treated with 329 ml of 5% sodium hypochlorite solution dropwise over 40 minutes while cooling to maintain the reaction temperature at < 30°. Stirring was continued for five hours at room temperature. Ten grams of sodium bisulfite was added in several portions over several minutes. After stirring overnight, the product was collected by

filtration and air-dried to give 33 g of a cream-colored powder which was crystallized from water to give analytically pure 6,9-dichloro-7,8-dihydroxy-1-phenyl-2,3,4,5-tetrahydro-1H-3-benzazepine, hydrochloride;. m.p. 185—200° (dec.).

Example 3

2-Chloro-3,4-dimethoxyphenethylamine (1.0 g) was reacted with 0.70 g of *p*-methoxystyrene oxide to give the hydroxyphenethylamine; m.p. 118.5—121°. This compound (2.16 g) was stirred at room temperature in 15 ml of trifluoroacetic acid with 4 drops of conc. sulfuric acid. Working up gave, after purification, on a silica gel column and using chloroform and 10% methanol/chloroform as eluates, the desired 6-chloro-7,8-dimethoxy-1-*p*-methoxyphenyl-2,3,4,5-tetrahydro-1H-3-benzazepine (0.,78 g), m.p. 143—145°.

This trimethoxy product (0.87 g, 2.50 mmoles) in 25 ml of dry methylene chloride was cooled in an ice-methanol bath as 12.5 ml (25.0 mmoles) of boron tribromide in methylene chloride were added dropwise. After stirring for 4 hours, the mixture was cooled in an ice bath while methanol was carefully added to give 0.37 g, after crystallization from methanol/ethylacetate, of 6-chloro-7,8-dihydroxy-1-*p*-hydroxyphenyl-2,3,4,5-tetrahydro-1H-3-benzazepine hydrobromide, m.p. 215°. Treatment with base and then methanesulfonic acid gave the methanesulfonate salt, m.p. 272°.

A solution of 402 mg (1.0 mmoles) of the methanesulfonate salt in 12 ml of methanol and 4 ml of conc. hydrochloric acid was treated with a solution of 251 mg (1.1 mmole) of 2,3-dichloro-5,6-dicyano-1,4-benzoqüinone (DDQ) in 2 ml of methanol at room temperature. After stirring overnight, the reaction mixture was treated with aqueous sodium bisulfite until the red color was discharged. The solution was concentrated *in vacuo* to about 1/2 volume. The tan precipitate was collected by filtration and air-dried to give 430 mg of 6,9-dichloro-7,8-dihydroxy-1-(*p*-hydroxyphenyl)-2,3,4,5-tetrahydro-1H-3-benzazepine, hydrochloride; m.p. 220—225° (dec.).

Example 4

Substituting a stoichiometric quantity of 2-fluoro-3,4-dimethoxyphenethylamine in the synthetic procedure of Example 3 gave 6-fluoro-7,8-dimethoxy-1-(*p*-methoxyphenyl)-2,3,4,5-tetrahydro-1H-3-benzazepine as a yellow oil. Hydrolysis with boron tribromide gave 6-fluoro-7,8-dihydroxy-1-(p-hydroxyphenyl)-2,3,4,5-tetrahydro-1H-3-benzazepine hydrobromide, m.p. 277° (dec.). DDQ and hydrochloric acid treatment as in Example 3 gives 9-chloro-6-fluoro-7,8-dihydroxy-1-*p*-hydroxyphenyl)-2,3,4,5-tetrahydro-1H-3-benzazepine hydrochloride.

Example 5

Submitting each of the following compounds to the hypochlorite treatment of Example 2 give the corresponding 6,9-dichloro compounds of this invention:

7,8 - dihydroxy - 1 - (3 - trifluoromethylphenyl) - 2,3,4,5 - tetrahydro - 1H - 3 - benzazepine hydrobromide gives 6,9 - dichloro - 7,8 - dihydroxy - 1 - (3 - trifluoromethylphenyl) - 2,3,4,5 - tetrahydro - 1H - 3 - benzazepine hydrochloride

7,8 - dihydroxy - 3 - ethyl - 1 - phenyl - 2,3,4,5 - tetrahydro - 1H - 3 - benzazepine hydrobromide gives 6,9 - dichloro - 7,8 - dihydroxy - 3 - ethyl - 1 - phenyl - 2,3,4,5 - tetrahydro - 1H - 3 - benzazepine hydrochloride

1 - (*b* - chlorophenyl) - 7,8 - dihydroxy - 2,3,4,5 - tetrahydro - 1H - 3 - benzazepine hydrobromide gives 6,9 - dichloro - 7,8 - dihydroxy - 1 - (*p* - chlorophenyl) - 2,3,4,5 - tetrahydro - 1H - 3 - benzazepine hydrochloride.

Example 6

A mixture of 4 g of 6,9-dichloro-7,8-dimethoxy-1-phenyl-2,3,4,5-tetrahydro-1H-3-benzazepine in 15 ml of formic acid and 10 ml of formaldehyde is refluxed for 18 hours. The mixture is evaporated *in vacuo*. The residue is treated with 20 ml of 6N hydrochloric acid. The solution is again eaporated to give a residue which is treated with 20 ml of 10% sodium hydroxide solution and then taken into ether. The ethereal extracts are dried and evaporated to give 6,9-dichloro-7,8-dimethoxy-3-methyl-1-phenyl-2,3,4,5-tetrahydro-1H-3-benzazepine.

This material (.13 g) in 75 ml of methylene chloride is treated with 3.2 g of boron tribromide at —10°. Working up gives 6,9-dichloro-7,8-dihydroxy-3-methyl-2,3,4,5-tetrahydro-1H-3-benzazepine hydrobromide.

An alternative N-methylation includes N-formylation of the 6,9-dichloro-7,8-dihydroxy compound of Example 2 with catalytic hydrogenation. For example, the starting material is reacted with formaldehyde in methylene chloride with palladium-on-charcoal under low pressure hydrogenation conditions. After absorption of hydrogen, the catalyst is removed, and the solvent is evaporated.

Substituting N-acylation with thenoyl chloride or $\alpha$-furylacetyl chloride followed by reduction gives the 3-thenyl or $\alpha$-furyl methyl compounds. Using allyl bromide with the dihydroxydichloro compound with an excess of potassium carbonate in acetone-methanol in the cold, then stirring at

room temperature, gives 3-allyl-6,9-dichloro-7,8-dihydroxy-1-phenyl-2,3,4,5-tetrahydro-1H-3-benzazepine hydrochloride.

Example 7

A solution of 540 g (containing 500 g) of commercial grade 4-methoxystyrene in 2 l. of methylene chloride was cooled to −10° and 521.2 g of bromine were added dropwise, keeping the temperature between −5° and −10°. The last drop caused an abrupt change in color, and the addition of bromine was stopped. The solvent was immediately removed under vacuum keeping the bath temperature below 35° and feeding the reaction solution in to the reaction vessel slowly. When the methylene chloride had been removed, the warm oil was mixed with 1600 ml (5.5 ml/g product) of hexane to disolve the remaining product in the evaporation flask and the bulk of the product. This required warming the mixture on a steam bath. Charcoal was added, the solution was filtered, and the resulting pale yellow solution was chilled in an ice bath to give 637 g (58.2% based on styrene; 66% based on bromine uptake) of crystals of 1-(4-methoxyphenyl)-1,2-dibromoethane m.p. 74—78°, the pure compound melts 80—81°.

About 100 ml of liquid was distilled from 600 ml of *t*-butyl alcohol. To the slightly cooled contents of the still pot were added 15 g of anhydrous powdered magnesium sulfate. The suspension was stirred for 15 minutes and then 100 g of the dibromoethane were added. The reaction mixture was refluxed with stirring for 1.5 hours. About 200 ml of methylene chloride were added to the cooled reaction mixture and the solids were removed by filtration. The solids were washed thoroughly with methylene chloride; the washings were combined with the filtrate and concentrated to dryness at 60°. The residue was stirred with 500 ml of pentane and 500 ml of water, and the layers were separated. The organic layer was washed with 5% sodium bicarbonate, dried and concentrated under vacuum to give 91.7 g (94%) of a tan oil; 2-bromo-1-(*t*-botoxy)-1-(4-methoxyphenyl)ethane.

2-(3,4-Dimethoxy-5-methylphenyl)ethylamine (43.1 g, 0.2 mole), the above bromoethane (57.4 g, 0.2 mole), sodium carbonate (25.2 g, 0.3 mole), and dimethylformamide are heated with stirring under nitrogen for 2 hours at 140°. The reaction mixture is poured into water, extracted with methylene chloride and the organic layer is washed twice with water. The organic layer is then washed twice with 1N aqueous hydrochloric acid, then with 10% aqueous sodium carbonate, and finally with saturated saline. Drying and concentrating the organic layer gives a brown oil which was treated with 400 ml of 10% sulfuric acid with vigorous stirring for 30 minutes. The reaction mixture after cooling is made basic with 40% sodium hydroxide, and then it is extracted twice with dichloromethane. The organic layer is washed with water, dried, and concentrated to give the desired N-[2-(3,4-dimethoxy-5-methylphenyl)ethyl]-2-(*p*-methoxyphenyl)-2-hydroxyethyl-amine.

The above substituted ethylamine (69 g, 0.20 mole) is dissolved in 520 ml of trifluoroacetic acid, and to the solution at 25° is added 29.4 g (0.30 mole) of conc. sulfuric acid, keeping the solution at 25°. The mixture is stirred at 25° for 3.5 hours, and then 74 g, 0.90 mole of sodium acetate are added with cooling. The reaction mixture is concentrated under a vacuum to remove the trifluoroacetic acid, water followed by ammonium hydroxide is added (pH 11), and the suspension is extracted with methylene chloride. Concentration gives a solid which is purified by recrystallization; 9-methyl-7,8-dimethoxy-1-(*p*-methoxyphenyl)-2,3,4,5-tetrahydro-1H-3-benzazepine.

A mixture of 21.6 (0.066 mole) of this trimethoxybenzazepine, 82.5 g (0.33 mole) of boron tribromide, and 216 ml of methylene chloride is allowed to react at 15° for 1 hour, and for 3 hours at 25°. Then 165 ml of methanol are added, keeping the reaction temperature at −20°. The temperature is slowly brought to 25°, and the slurry is concentrated under vacuum until most of the volatile material is gone. The residue is triturated with 100 ml of ethyl acetate, and the crystalline product is collected and washed with ether. This gives 9-methyl-7,8-dihydroxy-1-(*p*-hydroxyphenyl)-2,3,4,5-tetrahydro-1H-3-benzazepine hydrobromide. The free base is obtained by stirring with 2 moles of sodium bicarbonate in water, filtering, and washing well with water. It can be converted into the hydrochloride or methane sulfonate salts by treating a methanolic suspension of the free base with a 20% excess of the appropriate acid, adding excess ethyl acetate to precipitate the product, chilling, filtering, and then washing with ethyl acetate.

To a suspension of 32.2 g (0.1 mole) of the hydrochloride of the above benzazepine in 275 ml of methanol at 0° under argon is rapidly added a solution of 25.2 g (0.111 mole) of 2,3-dichloro-5,6-dicyano-1,4-benzoquinone. After stirring for 1 hour at 0°, the reaction mixture is filtered, and the solid is washed with 75 ml of cold methanol, 100 ml of ethyl acetate, and 100 ml of ether. The product is the 7,8-dione of the initial benzazepine. A stirred suspension of 4.8 g (0.015 mole of the 7,8-dione in 150 ml of methanol is treated with an excess of dry HCl. Concentration gives 1-(*p*-hydroxyphenyl)-7,8-dihydroxy-6-chloro-9-methyl-2,3,4,5-tetrahydro-1H-34-benzazepine hydrochloride which can be recrystallized from methanol-ethyl acetate.

1-Phenyl-7,8-dihydroxy-9-methyl-2,3,4,5-tetrahydro-1H-3-benzazepine is obtained by treating 2-(3,4-dimethoxy-5-methylphenyl)ethylamine in tetrahydrofuran with styrene oxide for 12 hours to give N-[(2-hydroxy-2-phenylethyl)]-2-(3′,4′-dimethoxy-5-methylphenyl which on cyclization gives the corresponding 7,8-dimethoxy benzazepine. Cleavage of this gives the desired 6-methyl compound as its hydrobromide which can be coverted via the free base into its hydrochloride.

Oxidation with DDQ in methanol with hydrochloric acid gives 6-chloro-9-methyl-1-phenyl-7,8-dihydroxy-2,3,4,5-tetrahydro-1H-3-benzazepine hydrochloride.

Example 8

Isovanillin (76.1 g, 0.5 m) was suspended in 750 ml of chloroform. Bromine (27.3 ml, 0.5 m) in 200 ml of chloroform was added slowly at 0°. Water was added to give the desired 2-bromo-3-hydroxy-4-methoxybenzaldehyde, m.p. 197—203°.

The aldehyde product (46.2 g, 0.2 mole) was dissolved in 300 ml of dry dimethylformamide, and 69.1 g of potassium carbonate were added. 28.4 ml (0.30 mole) of dimethylsulfate were added dropwise at room temperature. After the addition, the reaction mixture was heated on a steam bath for 10 minutes. 29 Ml of water were added dropwise and the reaction mixture was again heated for 5 minutes on the steam bath. The reaction mixture was then poured into ice water and the precipitate was collected, 2-bromo-3,4-dimethoxybenzaldehyde, m.p. 80—81.5°.

The dimethoxybenzaldehyde (10 g, 0.04 mole) was dissolved in 100 ml of ethanol, and 5 g (0.132 mole) of sodium borohydride were added. The reaction mixture was stirred for 1 hour. The reaction mixture was poured into water and extracted into methylene chloride to give the corresponding benzyl alcohol (m.p. 74—76.5°). This was converted into the benzyl chloride as a tan liquid, using benzene and conc. hydrochloric acid, and then into the corresponding benzyl cyanide, m.p. 48—55° using sodium cyanide in dimethylsulfoxide.

The benzyl cyanide (8.05 g, 0.315 mole) was dissolved in 70 ml of dry tetrahydrofuran, and then it was added slowly to 80 ml of 1 *M* boron trifluoride in tetrahydrofuran at 5°. After refluxing for 2 hours, the mixture was cooled, and 40 ml of methanol were added carefully. After refluxing shortly and standing overnight, the mixture was concentrated to give a tan oil. Dilute hydrochloric acid was added. The resulting material was washed with ether, filtered, and the filtrate was made basic with 40% sodium hydroxide. After extracting with ether, washing, drying and evaporating the extracts the desired phenethylamine was obtained as a viscous, light-yellow oil.

The phenethylamine (0.12 mole) is heated to 115° in an oil bath. Styrene oxide (14.4 g, 0.12 mole) is added, and the reaction mixture is heated for 1 hour. After cooling to ~30°, 2:1 petroleum ether/acetone is added to give N-[(2-hydroxy-2-phenylethyl)]-N-[2-(2′-bromo-3′,4-dimethoxyphenyl)-ethyl]amine.

This substituted phenethylamine (0.0445 mole) is dissolved in 60 ml of trifluoroacetic acid, and 4.05 ml of concentrated sulfuric acid are added. The reaction mixture is refluxed for 2 hours. After cooling, most of the trifluoroacetic acid is stripped off, and the residue is poured into water. It is made basic with 10% sodium hydroxide and extracted twice with ether. The ether is dried and evaporated to give 6-bromo-7,8-dimethoxy-1-phenyl-2,3,4,5-tetrahydro-1H-3-benzazepine.

Using this general procedure with various substituted styrene oxides having one or more methyl, methoxy, methylthio or trifluoromethyl groups gives the corresponding 6-bromo intermediates not readily prepared by direct bromination which are used for preparing the 6-alkyl compounds of this invention by the methods described hereinafter.

The bromo-7,8-dimethoxy compound (100 g) in a large excess of ethyl formate is heated at reflux for 10 hours. Evaporation *in vacuo* and purification by fractional recrystallization give the 3- or N-formyl derivative.

6-Bromo-3-formyl-7,8-dimethoxy-1-phenyl-2,3,4,5-tetrahydro-1H-3-benzazepine (82.6 g, 0.212 mole) was dissolved in 1500 ml of toluene and added to a mixture of 0.678 mole of n-butyl lithium, 250 ml of toluene and 250 ml of ether at −78°. After addition, the mixture was stirred for 10 minutes. N-Methylformanilide (86 g, 0.636 mole) was added to the mixture, followed by stirring at −78° for 1 hour. The cooling bath was removed, 500 ml of 10% hydrochloric acid and 250 ml of water were added to give the 6-formyl derivative as the hydrochloride salt, m.p. 209—210°, after standing overnight. This material was N-formylated in an excess of ethyl formate at reflux for 6 hours.

The 3,6-diformyl-7,8-dimethoxy product was reacted with a slight excess of ethyl lithium in ethyl ether at 0—5° to give 7,8-dimethoxy-6-$\alpha$-hydroxypropyl-1-phenyl-2,3,4,5-tetrahydro-1H-3-benzazepine.

A mixture of 7.5 g of 7,8-dimethoxy-6-$\alpha$-hydroxypropyl-1-phenyl-2,3,4,5-tetrahydro-1H-3-benzazepine in 500 ml of ethylformate was heated at reflux for 5 hours, and then worked up using medium pressure liquid chromatography to give the N-formyl derivative.

This material (4.9 g) in 150 ml of chloroform and 50 ml of concentrated hydrochloric acid was heated at reflux for 2 hours. A small amount of aldehyde by-product was removed by bisulfite extraction to give the 6-$\alpha$-chloropropyl compound.

This material (3.6 g) in dry dimethylsulfoxide was added dropwise to a solution of 1.07 (0.028 mole) of sodium borohydride in dry dimethylsulfoxide. After stirring at room temperature, the mixture was heated on the steam bath for several hours, and then poured into water. The product was taken into ethyl ether-ethyl acetate and purified by chromatography using methanol-chloroform to give 3-formyl-7,8-dimethoxy-6-propyl-1-phenyl-2,3,4,5-tetrahydro-1H-3-benzazepine.

This material (1.9 g 0.0054 mole) in 50 ml of ethanol and 10 ml of 40% sodium hydroxide was heated at reflux for 2 hours. After stripping, the residue was taken up in methylene chloride-water. The

combined organic layers were dried and evaporated to give the 7,8-dimethoxy compound which (1 g) was reacted with 1 ml of boron tribromide in dry methylene chloride for 3 hours. After stripping and cooling, the residue was treated with methanol. The methanol was taken off, and the residue was dissolved in hot water (20 ml). Evaporation and cooling gave 7,8-dihydroxy-6-*n*-propyl-1-phenyl-2,3,4,5-tetrahydro-1H-3-benzazepine hydrobromide, m.p. 226—229°.

The 6-propyl-7,8-dihydroxy compound is oxidized with DDQ to the 7,8-quinone, and then treated with hydrochloric acid to give 9-chloro-6-*n*-propyl-7,8-dihydroxy-1-phenyl-2,3,4,5-tetrahydro-1H-3-benzazepine hydrochloride.

Example 9

6-Formyl-7,8-dimethoxy-1-phenyl-2,3,4,5-tetrahydro-1H-3-benzazepine hydrochloride (6.3 g, 0.018 mole) from Example 8 was converted into the free base by aqueous alkali and methylene chloride. The organic layer was dried with magnesium sulfate and evaporated. The residue was refluxed in 500 ml of ethyl formate for 2 hours. The excess formate was distilled off, and the residue was dissolved in ethyl acetate and extracted once with dil. hydrochloric acid. Drying and evaporation left 3,6-diformyl-7,8-dimethoxy-1-phenyl-2,3,4,5-tetrahydro-1H-3-benzazepine.

This compound (6.1 g, 0.018 mole) was dissolved in 50 ml of isopropanol. Solid sodium borohydride was added slowly until 1.33 g (.036 mole) had been added. The reaction mixture was stirred at room temperature for 2 hours, and then it was worked up with the careful addition of water and then dil. hydrochloric acid to the cooled solution. When the solution was acidic, the isopropanol was stripped off. Water and ether were added to the residue to dissolve it. The ethereal layer was washed with bicarbonate, dried and evaporated to give oily 3-formyl-7,8-dimethoxy-6-hydroxymethyl-1-phenyl-2,3,4,5-tetrahydro-1H-3-benzazepine.

The oil (~4 g) was dissolved in 50 ml of ethanol and 10 ml of 40% sodium hydroxide were added. The reaction mixture was heated at reflux for 1—1/2 hours. The ethanol was stripped off, and the residue was dissolved in ether and water. The ether layer was washed again with water and dried. The solution was acidified with ethereal hydrogen chloride. The resulting solid was separated by decanting the supernatant liquors. The residue was crystallized from methanol-ethyl-acetate to give 6-methyl-7,8-dimethoxy-1-phenyl-2,3,4,5-tetrahydro-1H-3-benzazepine hydrochloride, m.p. 223—227°.

This dimethoxy compound (3.1 g, 0.0093 mole) was converted into the free base with alkali-methylene chloride. The organic layer was washed with water and dried, and then it was cooled to —15° at which time 3 ml of boron tribromide were added. The reaction mixture was stirred 3—1/2 hours at room temperature. The volatiles were stripped off, and, after cooling to —15°, methanol was added until the solid dissolved. The methanol was evaporated, and the residue was dissolved in boiling water. The solution was treated with activated charcoal and filtered while hot. Crystallization of the hot solution gave 1.2 g of 6-methyl-7,8-dihydroxy-1-phenyl-2,3,4,5-tetrahydro-1H-3-benzazepine hydrobromide, m.p. 160—163°. The base and methylsulfonate salt are prepared as described above.

This compound is oxidized to the 7,8-quinone with DDQ, and then the 7,8-dione is reacted with hydrochloric acid as above to give 9-chloro-6-methyl-7,8-dihydroxy-1-phenyl-2,3,4,5-tetrahydro-1H-3-benzazepine hydrochloride.

Example 10

A 4.0 g sample of 3-benzyl-6,9-dimethyl-7,8-dihydroxy-1-phenyl-2,3,4,5-tetrahydro-1H-3-benzazepine (prepared from the 3-unsubstituted benzazepine by reaction with benzyl bromide in the presence of potassium carbonate) is dissolved in 50 ml of acetic anhydride, and the solution is heated on a steam bath for one hour. The reaction mixture is cooled, ice-water is added, and the solution is evaporated to dryness. The residue is triturated with ethyl acetate, the solution washed with water, dried, and the solvent is removed *in vacuo* to leave an oil. The latter is dissolved in ether, and ethereal hydrogen chloride is added to precipitate 3-benzyl-6,9-dimethyl-7,8-diacetoxy-1-phenyl-2,3,4,5-tetrahydro-1H-3-benzazepine hydrochloride.

The diacetoxy compound prepared above (3.5 g) is dissolved in 100 ml of ethanol, and 1 g of 10% palladium-on-carbon is added. The mixture is hydrogenated in a Parr apparatus at 50° under 3.5 kg/cm$^2$ of hydrogen for one hour. The reaction mixture is filtered, and the filtrate is evaporated to give 6,9-dimethyl-7,8-diacetoxy-1-phenyl-2,3,4,5-tetrahydro-1H-3-benzazepine hydrochloride.

Alternatively, 6,9-dichloro-7,8-dihydroxy-1-phenyl-2,3,4,5-tetrahydro-1H-3-benzazepine hydrobromide (10 g) is dissolved in trifluoroacetic acid and reacted with a stoichiometric amount of acetyl chloride at room temperature. The next day the reaction mixture is evaporated, and the residue is recrystallized to give the desired diacetoxy derivative.

Substituting other alkanoyl anhydrides or chlorides gives various 7,8-alkanoyl derivatives.

Example 11

1,2,3,4-Tetrafluorobenzene (67.7 g), sodium hydrobromide (20.8 g), and 200 ml of ethyleneglycol are refluxed with good stirring for 3 hours. The reaction mixture is poured into water (800 ml), acidified with 10% hydrochloric acid, and then extracted with ether. The ether is washed with dilute sodium hydroxide and water, and then distilled under vacuum to give 2-(2,3,6-trifluorophenoxy)-ethanol, b.p. about 100° at 15 mm of mercury.

2-(2,3,6-Trifluorophenoxy)ethanol (48.8 g, 0.254 mole) and 24 g of anhydrous potassium carbonate in 800 ml of dimethylformamide were refluxed for 20 hours and then poured into water. This was extracted with ether, washed with water, and dried to give 5,8-difluoro-1,4-benzodioxane.

5,8-Difluoro-1,4-benzodioxane (30.6 g, 0.02 mole) is dissolved in 126 ml of acetic acid, 31.2 ml of 37% formaldehyde aqueous solution is added. Dry hydrogen chloride gas is bubbled in continuously, keeping the temperature between 35—38°. After 4 hours the reaction mixture is poured into water, extracted twice with ether, the extracts are washed with water, sodium bicarboante, and water, and then dried. Concentration gives 6-chloromethyl-5,8-difluoro-1,4-benzodioxane.

6-Chloromethyl-5,8-difluoro-1,4-benzodioxane (36.9 g, 0.183 mole) is dissolved in 500 ml of dimethylsulfoxide and 11.2 g (0.23 mole) of sodium cyanide are added. This mixture is stirred at 25° for 1 hour, and then poured into cold water. Extracting with ether, washing the extract with water, drying, and concentrating gives 6-cyanomethyl-5,8-difluoro-1,4-benzodioxane.

6-Cyanomethyl-5,8-difluoro-1,4-benzodioxane (30.34 g, 0.158 mole) dissolved in 300 ml of dry tetrahydrofuran is treated with 308 ml of 1 *M* borane in tetrahydrofuran under nitrogen. After 2 hours of reflux, 300 ml of methanol are added continuously (hydrogen evolution occurs, vigorously at first), then the mixture is refluxed for 30 minutes. Then 50 ml of 10% hydrochloric acid are added very carefully (hydrogen gas evolution again), and the mixture is refluxed for another 30 minutes. The reaction mixture is concentrated under vacuum to a small volume, then 300 ml of water are added, and the mixture is made basic with 40% sodium hydroxide. This is extracted with ether, dried, and concentrated to give 6-(2-aminoethyl)-5,8-difluoro-1,4-benzodioxane.

The primary amine (21 g), 2-bromo-1-(*t*-butoxy)-1-(4-methoxyphenyl)ethane (25 g), sodium carbonate (12.6 g) and dimethylformamide are mixed and heated at 140° for 3 hours. The mixture is poured into water and it is extracted with methylene chloride. The organic extracts are washed twice with water, then twice with 1N hydrochloric acid, 10% sodium carbonate, and finally with saturated saline. Drying the organic extract and concentrating *in vacuo* gives a residue which is treated with 200 ml of 10% sulfuric acid with stirring for 30 minutes. The mixture is cooled, made basic with 10% sodium hydroxide, and then extracted several times with dichloromethane. The combined organic layers are washed with water, dried, and evaporated to give the corresponding secondary amine.

This crude amine (27 g) is dissolved in 300 ml of trifluoroacetic acid, and a one-fold molar excess of concentrated sulfuric acid is added at 25°. After stirring for 3 hours, the mixture is mixed with an excess of sodium acetate with cooling. The mixture is concentrated *in vacuo* to remove the trifluoroacetic acid. Water is added to the residue, followed by ammonium hydroxide to pH 11. The suspension is extracted with methylene chloride. The combined extracts are washed, dried, and concentrated to give 6,9-difluoro-1-(*p*-methoxyphenyl)-7,8-dioxyethylene-2,3,4,5-tetrahydro-1H-3-benzazepine.

The ethyleneoxydifluorobenzazepine (12.7 g), 200 ml of 47% hydrogen iodide, and 5 ml of hypophosphorous acid are heated at reflux for 6 hours under nitrogen. Cooling gives a solid which is collected, and then washed with ethyl acetate and ether to give 6,9-difluoro-7,8-dihydroxy-1-(*p*-hydroxyphenyl)-2,3,4,5-tetrahydro-1H-3-benzazepine hydroiodide. This compound is converted into the base using sodium carbonate, and the hydrobromide salt is prepared as described above. This product was obtained as an amorphous film with consistent spectral data. The mass spectrum demonstrated P = 307.1.

Example 12

Using the hypochlorite addition reaction 9-chloro-7,8-dihydroxy-1-(*p*-hydroxyphenyl)-6-methyl-2,3,4,5-tetrahydro-1H-3-benzazepine hydrochloride, m.p. 213° was prepared.

Example 13

| Ingredients | Mg. per Capsule |
|---|---|
| 6,9-Dichloro-7,8-dihydroxy-1-(*p*-hydroxyphenyl)-2,3,4,5-tetrahydro-1H-3-benzazepine methanesulfonate | 50 (free base) |
| Magnesium stearate | 2 |
| Lactose | 200 |

The above ingredients are thoroughly mixed and placed into hard gelatin capsules. Such capsules are administered orally to subjects in need of treatment for from 2—5 times daily to induce peripheral dopaminergic activity to treat hypertension.

Example 14

| Ingredients | Mg. per Tablet |
|---|---|
| 9-Chloro-7,8-dihydroxy-6-methyl-1-(*p*-hydroxyphenyl)-2,3,4,5-tetrahydro-1H-3-benzazepine methanesulfonate | 100 (free base) |
| Corn starch | 30 |
| Polyvinyl pyrrolidone | 12 |
| Corn starch | 16 |
| Magnesium stearate | 3 |

The first two ingredients are thoroughly mixed and granulated. The granules obtained are dried, mixed with the remaining corn starch and magnesium stearate, and they are compressed into scored tablets which can optionally be broken in two for 50 mg dosages.

Sustained release capsules can be prepared using methods known to the art. Of course one such capsule can replace several conventional tablets or capsules.

**Claims**

1. A compound of the formula:

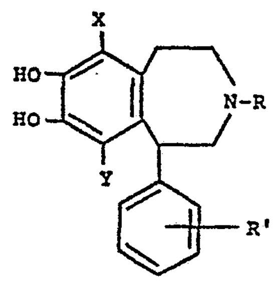

I

in which:

X and Y, which are the same of different, are each chloro, fluoro or lower alkyl of 1—4 carbons;

R is hydrogen, methyl, ethyl, allyl, dimethylallyl, thenyl or furylmethyl; and

R′ is hydrogen or one or two methyl, methoxy, hydroxy, acetoxy, halo, trifluoromethyl or methylthio substituents.

2. 6,9- Dichloro-7,8-dihydroxy-1-(*p*-hydroxyphenyl)-2,3,4,5-tetrahydro-1H-3-benzazepine.

3. 6-Methyl-9-chloro-7,8-dihydroxy-1-(*p*-hydroxyphenyl)-2,3,4,5-tetrahydro-1H-3-benzazepine.

4. 6,9-Dimethyl-7,8-dihydroxy-1-(*p*-hydroxyphenyl)-2,3,4,5-tetrahydro-1H-3-benzazepine.

5. A compound according to any of the preceding claims in the form of a pharmaceutically acceptable acid addition salt thereof or in the form of O-lower ($C_1$—$C_7$) alkyl or O-lower ($C_2$—$C_7$) alkanoyl derivative thereof.

6. A process for preparing a compound as claimed in claim 1, characterized in that a compound of the formula:

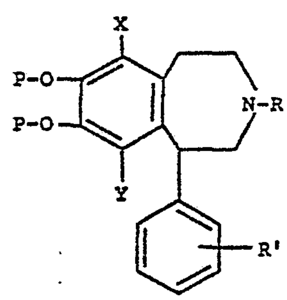

(in which X, Y, R and R′ defined in claim 1 but R′ may be O—P as well and P is an ether-forming protective group) is subjected to removal of the ether-forming protective group.

7. A process for preparing a compound as claimed in claim 1 where Y is chloro, characterized in that a compound of the formula:

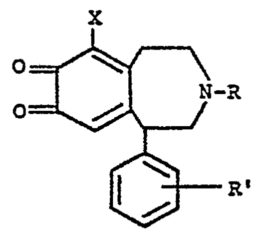

III

(in which X, R and R′ are as defined in claim 1) is reacted with HCl.

8. A process according to claim 6 or claim 7, characterized in that the compound of formula I produced is converted into a pharmaceutically acceptable acid addition salt thereof.

9. A pharmaceutical composition comprising a compound according to any one of claims 1 to 5 or as prepared by a process according to any one of claims 6 to 8 and a pharmaceutically acceptable carrier or diluent.

10. A composition according to claim 9 in dosage unit form.

**Patentansprüche**

1. Verbindung der Formel I

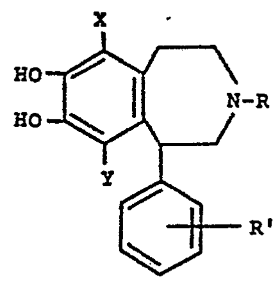

(I)

in der:

X und Y gleich oder verschieden sind und je ein Chlor- oder Fluoratom oder einen Niederalkylrest mit 1 bis 4 Kohlenstoffatomen bedeuten;

R ein Wasserstoffatom oder eine Methyl-, Äthyl-, Allyl-, Dimethylallyl-, Thenyl- oder Furylmethylgruppe bedeutet; und

R′ ein Wasserstoffatom oder ein oder zwei Methyl-, Hydroxy-, Methoxy-, Acetoxy-, Halogen-, Trifluoromethyl- oder Methylthiosubstituenten Bedeutet.

2. 6,9-Dichlor-7,8-dihydroxy-1-(p-hydroxyphenyl)-2,3,4,5-tetrahydro-1H-3-benzazepin.

3. 6-Methyl-9-chlor-7,8-dihydroxy-1-(p-hydroxyphenyl)-2,3,4,5-tetrahydro-1H-3-benzazepin.

4. 6,9-Dimethyl-7,8-dihydroxy-1-(p-hydroxyphenyl)-2,3,4,5-tetrahydro-1H-3-benzazepin.

5. Verbinding nach einem der vorstehenden Ansprüche in der Form ihres pharmazeutisch verträglichen Säureadditionssalzes oder in der Form eines O-nieder($C_1$—$C_7$)-alkyl oder O-nieder($C_2$—$C_7$)-alkanoylderivats.

6. Verfahren zur Herstellung einer Verbindung nach Anspruch 1, dadurch gekennzeichnet, daß aus einer Verbindung der Formel:

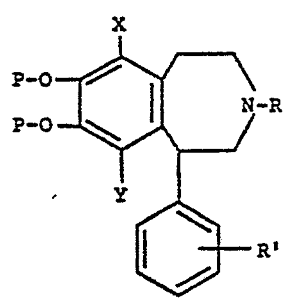

in der X, Y, R und R′ die in Anspruch 1 angegebene Bedeutung haben, R′ jedoch ebenso O—P bedeuten kann und P eine ätherbildende Schutzgruppe darstellt, die ätherbildende Schutzgruppe entfernt wird.

7. Verfahren zur Herstellung einer Verbindung nach Anspruch 1, in der Y ein Chloratom ist, dadurch gekennzeichnet, daß man eine Verbindung der Formel:

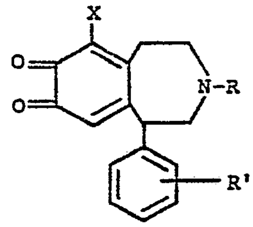

(III)

in der X, R und R′ die in Anspruch 1 angegebene Bedeutung haben, mit HCl umsetzt.

8. Verfahren nach Anspruch 6 oder 7, dadurch gekennzeichnet, daß man die erhaltene Verbindung der Formel I in ihr pharmazeutisch verträgliches Säureadditionssalz überführt.

9. Arzneimittel, enthaltend eine Verbindung nach einem der Ansprüche 1 bis 5, oder hergestellt durch ein Verfahren gemäß einem der Ansprüche 6 bis 8, und einen pharmazeutisch verträglichen Träger oder ein Verdünnungsmittel.

10. Arzneimittel nach Anspruch 9 in einer Dosiseinheit.

**Revendications**

1. Composé de formule:

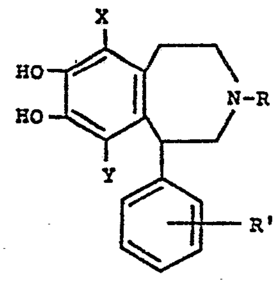

I

dans laquelle

X et Y, qui sont identiques ou différents, représentent chacun un radical chloro, fluoro ou alcoyle inférieur ayant de 1 à 4 atomes de carbone;

R représente un atome d'hydrogène, un groupe méthyle, éthyle, allyle, diméthylallyle, thényle ou furylméthyle; et

R′ représente un atome d'hydrogène ou un ou deux substituants méthyle, méthoxy, hydroxy, acétoxy, halo, trifluorométhyle ou méthylthio.

2. 6,9-Dichloro-7,8-dihydroxy-1-(*p*-hydroxyphényl)-2,3,4,5-tétrahydro-1H-3-benzazépine.

3. 6-Méthyl-9-chloro-7,8-dihydroxy-1-(p-hydroxyphényl)-2,3,4,5-tétrahydro-1H-3-benzazépine.

4. 6,9-Diméthyl-7,8-dihydroxy-1-(*p*-hydroxyphényl)-2,3,4,5-tétrahydro-1H-3-benzazépine.

5. Composé suivant l'une quelconque des revendications précédentes sous la forme de son sel d'addition d'acide pharmaceutiquement acceptable ou sous la forme de son dérivé O-alcoylé inférieur en $C_1$—$C_7$ ou O-alcanoylé inférieur en $C_2$—$C_7$.

6. Procédé de préparation d'un composé tel que revendiqué dans la revendication 1, caractérisé en ce qu'on soumet un composé de formule:

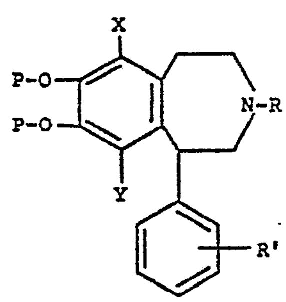

(dans laquelle X, Y, R et R′ sont tels que définis dans la revendication 1 mais R′ peut également être O—P et P représente un groupe protecteur formant éther) à l'élimination du groupe protecteur formant éther.

7. Procédé de préparation d'un composé tel que revendiqué dans la revendication 1 où Y représente un radical chloro, caractérisé en ce qu'on fait réagir un composé de formule:

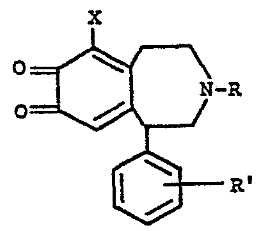

III

(dans laquelle X, R et R' sont tels que définis dans la revendication 1) avec HCl.

8. Procédé suivant la revendication 6 ou la revendication 7, caractérisé en ce que l'on convertit le composé de formule I engendré en son sel d'addition d'acide pharmaceutiquement acceptable.

9. Composition pharmaceutique comprenant un composé suivant l'une quelconque des revendications 1 à 5 ou tel que préparé par un procédé suivant l'une quelconque des revendications 6 à 8 associé à un excipient ou diluant pharmaceutiquement acceptable.

10. Composition suivant la revendication 9 sous forme de posologie unitaire.